# EUROPEAN PATENT SPECIFICATION

(11) **EP 4 103 686 B1**
(45) Date of publication and mention of the grant of the patent: **23.08.2023**
(21) Application number: 21705922.9
(22) Date of filing: 12.02.2021
(51) Int. Cl.: C12N 1/20, C12N 15/01, C12P 5/02

(54) **BIOCATALYST ADAPTATION AS LOAD FOLLOWING SOLUTION**
BIOKATALYSATORANPASSUNG ALS LAST NACH EINER LÖSUNG
ADAPTATION DE BIOCATALYSEUR EN TANT QUE SOLUTION DE SUIVI DE CHARGE

(30) Priority: 13.02.2020 DE 102020103803
(43) Date of publication of application: 21.12.2022
(60) Divisional application: 23185017.3
(73) Proprietor: ELECTROCHAEA GMBH, 82152 Planegg (DE)
(72) Inventor: COCIANCICH, Matteo, 80339 Munich (DE); PINDER, Zachary, 81379 Munich (DE); LARDON, Laurent, 4320 Lejre (DK); HAFENBRADL, Doris, 82049 Pullach (DE)
(74) Representative: Fleuchaus & Gallo Partnerschaft mbB
(86) International application number: PCT/EP2021/053472
(87) International publication number: WO 2021/160811

(56) References cited:
- EP-A1- 3 072 979
- WO-A1-2008/094282
- J-P. STEYER ET AL: "Advanced control of anaerobic digestion processes through disturbances monitoring", WATER RESEARCH, ELSEVIER, AMSTERDAM, NL, vol. 33, no. 9, June 1999 (1999-06), pages 2059-2068, XP004165765, ISSN: 0043-1354, DOI: 10.1016/S0043-1354(98)00430-8

## Description

The present invention refers to an improvement in stabilizing the biological methanogenesis process by biocatalysts, e.g., Archaea. In particular, the present invention refers to methods for adapting and stabilizing the biocatalyst and the methanogenesis process. More particularly, this method provides an improvement for and during unstable or excessive fluctuation in the energy load and thereupon fluctuations regarding the supply of feed gas.

Parts of the project leading to this invention have received funding from the European Union's Horizon 2020 research and innovation program under the grant agreement No. 691797.

The pursuit of efficient energy supplying solutions at a lower cost for the environment is a challenge set forth by climate action programs all over the world. The ultimate goal is to fulfill the increasing need for sustainable and renewable energy while counteracting climate change towards the transition to a modern, CO₂ balanced economy. Methane has the highest energy density per carbon atom among volatile hydrocarbons and its potential for energy conversion is far greater than any other natural gas. Therefore, methane constitutes a sustainable and renewable energy source and already today increasingly substitutes coal and other fossil fuels.

As methane's potential for energy generation has become increasingly relevant, recent developments have focused on the methods for producing methane by using methanogens, e.g., Archaea, which are capable of producing very efficiently renewable methane from carbon dioxide and hydrogen. Presently, the first large scale reactors applying this technology are established and running to enrich gas compositions with methane produced by employing methanogenic microorganisms.

EP 2661511, EP 3072979 and EP2032709 describe well the general method, suitable reactor designs and methanogenic microorganisms that may be used for biological methanogenesis.

With the prerequisite of an availability of substrate, primarily the carbon dioxide and electrolysis generated hydrogen, which can be used as feeding gases for the employed methanogenic microorganisms, this methane production method can be established in suitable bioreactors/cells all over the world.

In 2020 we are aware of about 15 mainly non-commercial pilot plants in Europe, which use various approaches to exploit microorganisms to generate methane by biological methanogenesis in suitable reactors.

Due to its promising potential, the large-scale utilization of biomethane is under attentive political and economic scrutiny to render the technology remunerative and cost-effective, and harnessing methane has been identified as the most important near-term goal for biochemical engineering.

However, despite a tremendous flexibility and variability of this methodology, it should be understood that this technology, which is often labeled "Power-to-Gas", can only become economically and commercially viable when the tremendous need of energy for e.g., the hydrolysis and generation of hydrogen can be satisfied from renewable or green energy sources.

The main disadvantage of renewable or green energy sources is their unpredictability and their lack of consistency. With an inconsistent influx of wind energy or solar energy also the generation of hydrogen lacks in consistency and makes storage of a hydrogen supply necessary, in order to guarantee access to a constant and adaptable amount of hydrogen as feeding gas.

Additionally, also the second feeding gas, CO₂, may originate from sources with a variable output, e.g., biogas plants. The risk of variable amounts of CO₂ influx has also an impact on the stability of the bio-methanogenesis process, in particular on the growth and methane production rate of the biocatalyst, e.g., the methanogenic microorganisms.

While on a laboratory scale such problems are virtually non-existent or can be easily compensated by regulating the feeding gas flow, in large scale production units they typically cause a drop, a crash or at least a measurable inconsistency in the methane production and product gas quality. As a consequence of such instable production process the product gas may contain a too high amount of feeding gases and/or may not contain the required amount of methane for further utilization. This interrupts a continuous flow of grid-suitable product gas and requires the additional employment of either cost-intensive gas purification or enrichment steps until the product gas satisfies the typical grid requirements. In any case the consequence is a loss in effective production time and thus, a financial disadvantage.

It is thus an object of the present invention to improve the methodology of large-scale bio-methanation processes and to teach process modifications which guarantee a reliable production of grid satisfying methane despite any disruption or variation in the gas feed and/or a disruption or variation regarding the energy supply.

The solutions hereby described are applicable for the improvement of primarily large-scale bio methanation processes using hydrogenotrophic methanogens and in particular archaea, which can use hydrogen for reducing organic components, e.g., CO₂, formate, methanol or others, to methane. Bio-methanation processes using hydrogenotrophic methanogens have been described in literature [Martin *et al*., 2013; Thauer *et al*., 2008]. In particular, CO₂ may be reduced according to the following stoichiometric equation:

CO₂ + 4 H₂ → CH₄ + 2 H₂O.

In an industrial plant, this stochiometric equation is implemented by a complex energy supply system, which comprises and/or combines:
- a H₂ gas feed, wherein the H₂ is preferably generated in a reactor from H₂O by means of a suitable electrolysis reaction that uses electric energy, the electric energy preferably deriving from or being generated by renewable energy sources;
- a CO₂ gas feed, wherein, for instance, the CO₂ is generated by a biogas reactor or is an industrial waste gas;
- a methanation reaction in an aqueous medium, the methanation reaction being carried out in a suitable reactor by suitable methanogenic microorganisms which, in particular, convert the feeding gases H₂ and CO₂ in a ratio 4:1 to methane; and
- a product methane-containing gas collection unit.

The aforementioned lack of consistency in energy and/or gas flow turns out to be a major problem when establishing industrial plants for bio-methanation processes employing renewable energy sources and/or feeding gas from natural sources or biogas plants. Typically, this lack of consistency leads to a decrease in the methane production by the biocatalyst and/or in the quality of the product gas.

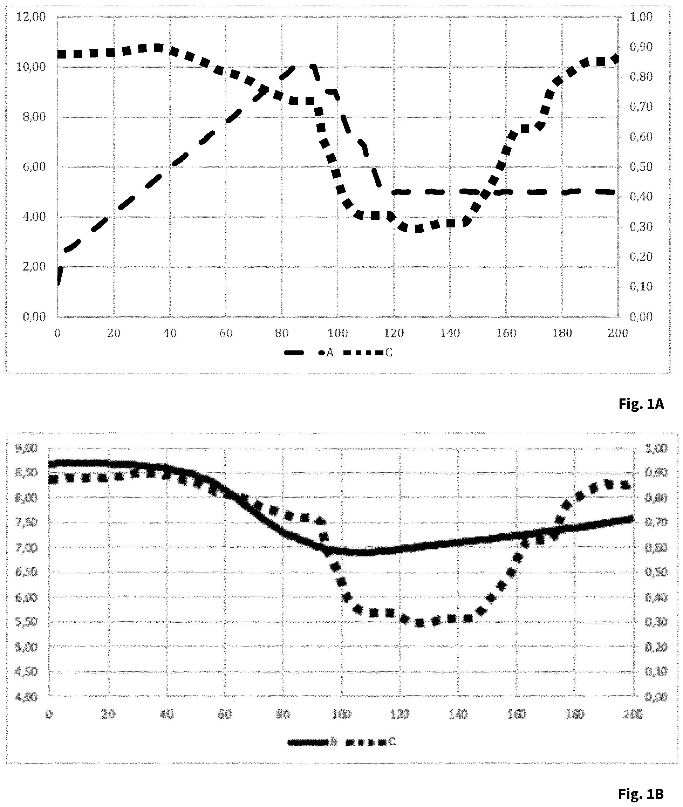

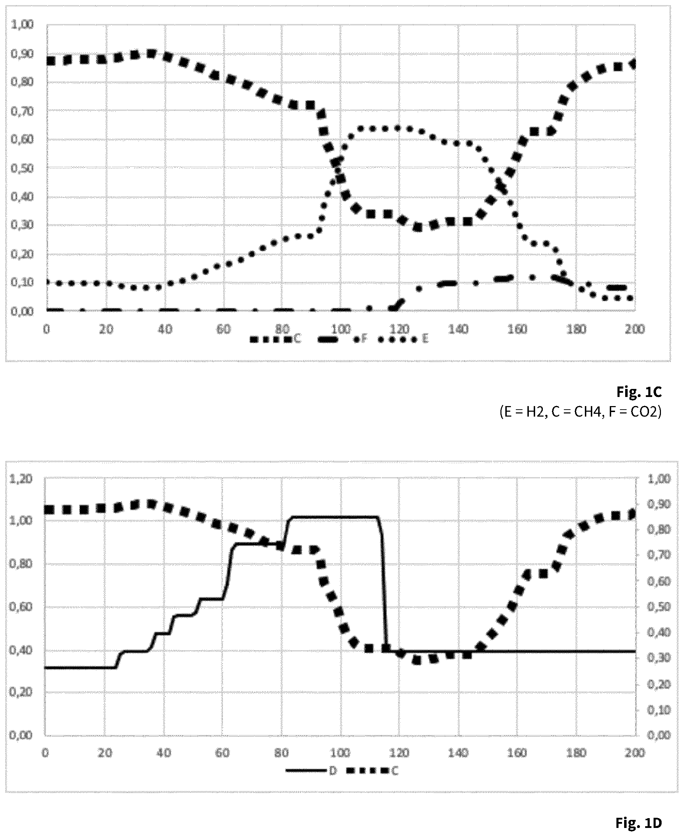

**Figure 1** data showing the impact of a typical disturbance caused by a lack of consistency in the feed gas flow rate and ammonia delivery and a representative timeframe for recovery from such disturbances in an existing industrial plant.

Fig. 1A depicts an exemplary variation of gas flow rate over time as represented by hydrogen flow in kg/h. For the entire timeframe depicted in Fig 1A the stoichiometric ratio of the feed gases (H₂ and CO₂) is held constant. Due to an external factor, the flow rate of the feed gases increases to a maximum level, is subsequently reduced over time and then is held constant until the end of the timeframe. The impact of these flowrate changes on product gas composition is depicted in Fig. 1A, 1B, 1C and 1D. In Fig. 1A the impact is illustrated as the changing volumetric fraction of methane in the product gas over the entire timeframe. This measure of product gas composition is used as a reference indicator of the reactor performance in each of the graphs for Figure 1. Fig. 1B depicts the pH value of the aqueous medium, illustrating acidification of the reactor media from pH 8.6 at the beginning of the time frame to below pH 7.0, and then recovery to a pH above 7.0 at the end of timeframe. As shown in Fig. 1C, there is no change in the methane, hydrogen or CO₂ composition in the product gas until the H₂ feed gas flow rate reaches 5kg/h (shown in Fig. 1A), after which the composition of the product gas declines to as low as 30%, eventually recovering to nearly 90% of the product gas, as was achieved for the product gas quality at the beginning of the timeframe. The variability in gas composition, including hydrogen, methane and CO₂ composition, during the changing flow rates and recovery to a stable condition are exemplary of the impact of disturbances due to fluctuations in gas flows or energy supply for methanation. Fig. 1D illustrates the state-of-the-art load following NH₄OH feeding rate which correlates to the increase in feed gas flows. The increase of the dosing following the increase in gas flow is clearly not enough to sustain acceptable methane content in the product gas, nor pH stability when cross referenced with Fig. 1B.

Typically, in order to attain an acceptable methane content stability and to decrease the amount of CO₂ and H₂ contained in the product gas, the methanation process needs to be re-adjusted by e.g. increasing the available nutrients such as ammonium content, adjusting the feed gas composition and/or by adjusting the feeding gas flow, thereby also recovering the pH value, cf. e.g., Fig. 1A and B.

Typically, the time interval between the appearance of a disturbance and the re-establishment of an acceptable methane production rate may range from about 2 to about 5 hours, e.g., depending on the extent of the disturbance. At least during this time interval, the product gas cannot be directly used and e.g., must be purified before it may be introduced in the gas grid.

In the context of the present application, the term "disturbance" refers to any external or internal change to an energy supply system for bio-methanation, said change reverberating on the gas feed, e.g., on the CO₂ and/or H₂ content. For example, the change to the energy supply system may lead to a change in the CO₂ and/or H₂ content of the gas feed, said change will cause a change in the given stoichiometric or any other ratio and as a consequence cause an interruption of a continuous methane production rate and/or a drop regarding the quality of the product gas. In particular, the term "disturbance" refers to any variations in electricity supply for a bio-methanation plant caused by natural phenomena, such as weather conditions, or other supply restrictions. Eventually, the term "disturbance" of a bio-methanation process is also understood as the man-caused interruption of the production process, e.g., for maintenance and/or cleaning purposes of the reactor.

The present invention provides a method for adapting the biocatalyst. Biocatalysts, e.g., Archaea adapted according to the method of the present invention turns out to be more resilient against disturbances or fluctuations in the gas feed volume. In particular, the methane content in the product gas of a bio methanation process that uses those adapted biocatalysts are - if at all - marginally affected by the occurrence of disturbances or fluctuations in the gas feed rate.

This way, readjustments of this bio-methanation process are superfluous, as this process does not experience a substantial decrease in the methane content at high gas flows and maintains a substantially stable methane content and a grid-quality product gas even when exposed to substantial disturbances or fluctuations in the gas feed.

In particular, the method of the present invention allows for stabilizing the methane production and the quality of the product gas against fluctuations in the gas feed volume of the order 15-20% and more. In an industrial-scale plant, such fluctuations may be caused by a disturbance and, in the case of conventional bio methanation processes, lead to a decrease in the methane content in the product gas that cannot be promptly compensated.

In particular, if a change, e.g., an increase or drop of 20% or more in the energy supply and/or in the corresponding gas feed is expected, bio-methanation plants known in the art has to be put in non-productive mode, e.g., shut down until the methane content at the required gas flow rate is stabilized and the product gas attains grid-quality.

With the method of the present invention such interruptions can be avoided by adapting the biocatalyst according to the method of the present invention prior to the expected change. In particular, a bio methanation process that uses the method of adapting the biocatalyst and consequently uses such adapted biocatalyst can handle an increase in the energy supply and/or in the corresponding gas feed of about 20%, 30%, 40%, 50%, 60%, and up to 100%, 130%, 150%, and even 200%.

In the context of the present invention, the term "methane production rate" or "MPR" is defined as the total amount of methane produced in the system in nominal point as m3/h (Thema et al., Energies 2019,12,1670). Additionally, according to the present invention, the term "methane content" in the product gas is measured as a volumetric fraction of the product gas. This "methane content" is kept stable as one of the major advantages of the present method and comprises a methane content of around and above 94%, 95%, 96%, 97%, 98% or 99% methane. This way, readjustments of this bio-methanation process become superfluous; because when applying the method of the invention the methanation process is not prone to any substantial decrease in the methane content of the product gas even at high gas flows and maintains a substantially stable methane content and a grid-quality product gas. In other words, the "methane content stability" as here described is not only an advantage but of high commercial interest, as even with instable or inconsistent energy or feed gas supply a bio-methanation plant applying the method of the present invention can avoid or at least minimize inefficient production times or production times where the product gas does not satisfy the grid quality requirements.

Moreover, in the context of the present invention, the term "grid-quality" is defined as the product gas quality which is required to inject methane gas into the gas grid. The quality requirements are defined by the gas grid operator and typically includes the requirement to contain at least 95% pure methane in the product gas.

The method for adaptation of the biocatalyst according to claim 1 teaches a biocatalyst conditioning (short term) and/or a biocatalyst adaptation (long term) to address any disturbance of the methanation process. The biocatalyst adaptation can be a physical and/or chemical adaptation. The order of starting and/or applying the adaptation to the biocatalyst the selection and order of steps may vary. Accordingly, it is possible to adapt the biocatalyst by either chemical or physical adaptation as well as a combination of chemical and physical adaptation and *vice versa.*

An advantage of the method according to the present invention is that bio-methanation processes using adapted biocatalysts prior to a variation in the gas feed flows which also maintain a stable feed gas ratio, guarantees a substantially stable methane content and a grid-quality product gas. A commercially interesting consequence is that an energy supply system and/or plant carrying out the method of the present invention has reduced need of additional and expensive purification systems but may use them for optional or voluntary improvements only, or not at all.

According to the invention, the biocatalyst may be challenged and forced into adaptation by administrating at least one selected metabolic enhancer to the aqueous culture medium. In particular, the biocatalyst may be challenged and forced into adaptation by adding a metabolic enhancer to the aqueous culture medium. In the context of the present invention, this addition of the metabolic enhancer is also referred to as "**chemical adaptation**". According to some embodiments, the chemical adaptation of the biocatalyst comprises either an individual or a repeated dosing of the metabolic enhancer into the aqueous culture medium.

In the understanding of this invention, it is important to distinguish the chemical adaptation form any previously described increase of the biomass. While it is known that the increase of available nitrogen in the reaction vessel may induce an increase in biomass concentration, the method of the present invention does not lead to an increase in biomass, but quite to the contrary, increase only the metabolic activity of the biocatalyst without increasing the growth rate or the biomass production. Additionally, and against the presumption that the amount of biomass is correlated with the MPR, it was found by the inventors that with the method of chemical adaptation as herein described the metabolism of the biocatalyst and thus, methane production can be stimulated without increasing the biomass as measured and controllable by the OD in the reaction vessel.

According to the present invention the method of the chemical adaptation is applied to a reaction vessel, which is already in operative methane production modus and which needs to be adapted to an expected feeding gas disturbance. Typically, in a methane production modus the amount of N in the culture vessel can be kept around 200-750 mg/l N; any additional investment of N or other ammonium sources is not recommended as an increase of the available N source will lead to an increase in biomass, which is unwanted and might cause instability until the amount of biocatalyst and the feeding rate of all necessary nutrients has been adapted again.

Accordingly, in further embodiments of the present invention the optical density of the biocatalyst in the reaction vessel measured at OD610 is preferably around OD 20-50. However, the biocatalyst can be cultured also at lower OD610, namely OD 10-20 or even OD610 of above OD 50 and up to OD 80. Preferably, the optical density of the biocatalyst is stable and remains unchanged prior to the preloading, during the preloading and even hours after the preloading and the increase in feeding gas.

However, and against such expectations, the present invention shows that an additional dosing of a metabolic enhancer, wherein this dosing is carried out prior to an expected disturbance of the feeding gas supply can stabilize the methane production even if the feeding gas supply is irregularly modulated up and/or down.

In other words, the method of chemical adaptation is a preloading with a metabolic enhancer. As the reaction time of the biocatalyst to nutrients, e.g. to the metabolic enhancer, is very fast, the dosing of, e.g. the preloading with the metabolic enhancer is effective when applied seconds or minutes before a feeding gas disturbance. The effect of the metabolic enhancer is believed to be a metabolic induction and may be easily analyzed by measuring and following the methane content in the product gas, e.g. the time dependence thereof. In particular, the methane content in the product gas will remain stable and substantially undisturbed over time e.g. despite the disturbance in e.g. the gas feed.

According to an embodiment of the present invention, the preloading with one or more metabolic enhancers into the aqueous culture medium in the methanation reactor is started prior to the disturbance. For example, the preloading may be initiated and/or carried out at least seconds before the disturbance, but also minutes or hours before the disturbance. For instance, the preloading according to the invention may be started at least 1 hour, 30 min, 15 min, 10 min, 5 min or 1 min prior to the expected disturbance.

According to some embodiments of the present invention, the preloading with the metabolic enhancer may be started several hours before the expected disturbance. For instance, the preloading according to the invention may be started at least 3 hours, 2 hours, 1 hour or 0,5 hour prior to the expected disturbance.

According to the invention and suitable with any of the above-described embodiments the dosing may be applied in a singular dose or alternatively, a plurality of doses.

According to the present invention, suitable metabolic enhancers are selected from the group of redox active chemicals comprising amino acids such as e.g. cysteine, which has a redox potential of -0.22V, carbonate solutions, pH changing solutions such as acids and bases, and preferably Na₂S, NH₄OH as well as combinations of any or several of these.

As described in Example 1 for the preloading with NH₄OH, the preloading may be started 0,5 hours prior to the disturbance. In particular, the preloading may be carried out by introducing a plurality of doses of NH₄OH, each dose of this plurality of doses being introduced at a respective point in time into the aqueous culture medium. Furthermore, in Example 1

Ultimately according to the method of the invention, either an injected single dose or the plurality of doses of NH₄OH, may typically sum up to an addition of about 5 to 25 mol -N, which is to be understood as being additionally available as target concentration in the culture medium. Alternatively, in other embodiments the additionally provided amount of nitrogen available in the culture medium can be 8 to 20 mol-N, further 5 to 10 mol-N, further 5 to 21 mol-N, further 8 to 21 mol -N, further 8 to 25 mol -N, further 10 to 25 mol -N, further 10 to 21 mol -N, or further 5 to 15 mol - N.

Alternatively, e.g. in case of a smaller time window between the detection of the expected perturbance and the occurrence of said disturbance, the preloading may be carried out by introducing a single dose of NH₄OH in the aqueous culture medium shortly prior to the disturbance. In particular, in an embodiment where a single dose is preferred this single dose of NH₄OH can amount to an addition of 1,5 to 21 mol -N in the culture medium. These absolute numbers are measured for a 1MW system, thus a reactor which has a methane production capacity of 1 MW (megawatt).

As illustrated in Example 1, the preloading with nitrogen according to the invention does not follow the CO₂ increase, but is given prior to the gas increase and thus anticipating an CO₂ increase.

While in the prior art reactor the load following doses of e.g. NH₄OH or any other nitrogen equivalent depends on the influx of CO₂ in Nm3 going into the system, and typically an amount of Y ml NH₄OH are added for an influx of X Nm3 CO₂, according to the method of the present invention the relevant amount of Y NH₄OH or any comparable nitrogen equivalent are preloaded in anticipation of X Nm3 CO₂. The amounts Y and X depend on the biocatalyst in use in a plant and are typically adjusted to the reactor volume and methanation rate of the biocatalyst, they should not be understood as absolute values. Furthermore, it is to note that any absolute numbers of nitrogen addition as given above may vary or will increase with an increasing size of the methane production system, e.g. any systems of 10 MW, 100MW or 200 MW production capacity. Consequently. the actual amount and timing of the preloading may be adjusted based on other operational parameters of the system, including mixing energy, pH, oxidative-reductive potential, temperature, pressure, ionic strength of the medium, or gas flow rate.

One advantageous effect of the preloading with the metabolic enhancer is that the biocatalyst is stimulated into a higher metabolism and thus capable to react much quicker to a sudden increase in nutrients such as reactant gases and eventually use the provided reactant gas more efficient to build methane. As a by-product, a methanation culture which has been treated according to the method of the invention and e.g., preloaded with a redox active chemical seems to be less sensitive to variations of the pH value of the aqueous culture medium.

Interestingly, it has been found that the stability of the pH value also is a further indicator for the successful adaptation of the biocatalyst. In other words, after applying the method of the present invention and thus, after adaptation of the biocatalyst, the stability of the pH value, despite substantial increase of the reactant gas CO₂, is a clear indicator of a successful adaptation and the additionally acquired potential to allow immediately increasing the methane production by the biocatalyst.

As a consequence, it is possible to measure a stable methane production prior, during, and after the disturbance, which, in particular, may be a reduction or an increase in the energy supply, which result in a corresponding change or increase in the feeding gas supply.

Beside the chemical adaptation, the biocatalyst may also be adapted to a disturbing event, e.g., a reduction or an increase in the energy supply and correlated changes in the feeding gas supply, by a so-called "**physical adaptation**".

According to the present invention, physically adapting the biocatalyst may comprise or consist of challenging the biocatalyst by forced changes in the gas feed prior to an expected disturbance. According to the present invention, physically adapting the biocatalyst may comprise ramping the flow of the feeding gas up and/or stressing the flow of the feeding gas.

Additionally, or in conjunction with the above, physically adapting the biocatalyst may comprises using of one of more physical means to increase the amount of reactant gases solved into the culture medium. In particular, physically adapting the biocatalyst comprises increasing the pressure of the reactor and the gas influx and/or increasing the stirring activity in the reactor.

"Ramping" the flow of the feeding gas up and/or "stressing" the flow of the feeding gas may be understood in the context of the present invention as comprising an increase of the flow rate of the feeding gas or a rapid decrease of the flow rate of the feeding gas. According to some embodiments, increasing the flow rate of the feeding gas may comprise, e. g. consist of, linearly and/or stepwise increasing of the flow rate of the feeding gas. Moreover, increasing the flow rate of the feeding gas may be a predetermined increasing, e.g., increasing the flow rate of the feeding gas from a first given value to a second given value in a predetermined amount of time.

According to an embodiment of the present invention, a forced change in the gas feed may be a single "on/off" interval, i.e., a single spike or pulse of the feeding gas to which the biocatalyst will react with a metabolic stimulation, which is considered already the first step of the adaptation.

According to the present invention, this single spike, and also a repetition of single spikes is considered individual steps of physical adapting the biocatalyst and can be carried out prior to the expected disturbance. It may start 5h, 4h, 3h or 2h, and preferably around 1h or 0,5h prior to the expected disturbance and/or wanted increase in feeding gas.

According to some embodiments of the present invention, stressing the flow of the feeding gas starts if the amount of methane in the product gas drops below a threshold value, e.g., 95%, and stops when the amount of methane in the product gas exceeds the threshold.

According to a further embodiment of the present invention, stressing the flow of feeding gas comprises:
i) increasing the flow of the feeding gas from a first value of the feeding gas flow to a second value of the feeding gas flow;
ii) maintaining the flow of the feeding gas at substantially the second value of feeding gas flow for a first amount of time; and
iii) after maintaining the flow of the feeding gas at substantially the second value of feeding gas flow, decreasing the flow of the feeding gas to a third value of the feeding gas flow.

In particular, the third value of the feeding gas flow is greater than or equal to the first value of feeding gas flow.

The third value of the feeding gas flow may be substantially equal to the first value of feeding gas flow. For example, the first value of the feeding gas flow and/or the third value of the feeding gas flow are comprised between A1 and A2, in particular between A3 and A4, more particularly between A5 and A6. Moreover, the second value of the feeding gas flow may be comprised between B1 and B2, in particular between B3 and B4, more particularly between B5 and B6.

In particular, the second value of the feeding gas flow may depend on, e.g., be proportional to, the first value of the feeding gas flow. For instance, the second value, *V*₂, of the feeding gas flow may be proportional to the first value, *V*₁, of the feeding gas flow, i.e., *V*₂ = *k̅* × *V*₁*.* The proportionality constant, *k̅*, may be comprised between 1,13 and 1,25, in particular between 1,15 and 1,23 and, more particularly, between 1,18 and 1,21. In particular, said proportionality constant may be equal to 1,20.

In particular, step ii) is carried out after e.g., immediately after, step i). Alternatively, or in conjunction with the above, step iii) may be carried out immediately after step ii).

Increasing the flow of the feeding gas from the first value to the second value may be carried out by linearly of stepwise increasing the flow of the feeding gas in a fifth amount of time. Alternatively, or in conjunction with the above, decreasing the flow of the feeding gas to the third value may be carried out by linearly or stepwise decreasing the flow of the feeding gas, e.g., from the second value to the third value. The fifth amount of time and/or the sixth amount of time may be much smaller than the first amount of time. For example, the fifth amount of time and/or the sixth amount of time are less than or equal to a tenth, a hundredth and/or a thousandth of the first amount of time.

In some embodiments of the method of the present invention, stressing the flow of the feeding gas further comprises:
iv) maintaining the flow of the feeding gas at substantially the third value of feeding gas flow for a second amount of time;
v) after maintaining the flow of the feeding gas at substantially the third value, increasing the flow of the feeding gas to a fourth value of the feeding gas flow;
vi) maintaining the flow of the feeding gas at substantially the fourth value of feeding gas flow for a third amount of time; and
vii) after maintaining the flow of the feeding gas at substantially the fourth value, decreasing the flow of the feeding gas to a fifth value of feeding gas flow.

In particular, the fifth value of the feeding gas flow is greater than or equal to the third value of feeding gas flow, the fourth value of the feeding gas flow is greater than or equal to the second value of feeding gas flow, and/or the third amount of time is greater than or equal to the first amount of time.

The fifth value of the feeding gas flow may be substantially equal to the third value of feeding gas flow and/or the fourth value of the feeding gas flow may be substantially equal to the second value of feeding gas flow. For example, the fifth value of the feeding gas flow is comprised between A1 and A2, in particular between A3 and A4, more particularly between A5 and A6. Moreover, the fourth value of the feeding gas flow may be comprised between B1 and B2, in particular between B3 and B4, more particularly between B5 and B6.

In particular, the fourth value of the feeding gas flow may depend on, e.g., be proportional to, the third value of the feeding gas flow. For instance, the fourth value, *V*₄, of the feeding gas flow may be equal to the product between the aforementioned proportionality constant, *k̅*, and the third value, *V*₃, of the feeding gas flow, i.e., *V*₄ = *k̅* × *V*₃.

The third amount of time may be substantially equal to the first amount of time. For example, the third amount of time and/or the first amount of time are greater than or equal to C1 and less than or equal to C2, in particular greater than or equal to C3 and less than or equal to C4, more particularly greater than or equal to C5 and less than or equal to C6.

In particular, step iv) is carried out after (e.g., immediately after) step iii), step v) is carried out immediately after step iv), step vi) is carried out after (e.g., immediately after) step v), and/or step vii) is carried out immediately after step vi).

Increasing the flow of the feeding gas to the fourth value may be carried out by linearly or stepwise increasing the flow of the feeding in a seventh amount of time e.g., from the third value to the fourth value of the feeding gas flow. Alternatively, or in conjunction with the above, decreasing the flow of the feeding gas to the fifth value may be carried out by linearly or stepwise decreasing the flow of the feeding gas in an eighth amount of time e.g., from the fourth value to the fifth value of the feeding gas flow. The seventh amount of time and/or the eighth amount of time may be much smaller than the third amount of time. For example, the seventh amount of time and/or the eighth amount of time are less than or equal to a tenth, a hundredth and/or a thousandth of the third amount of time.

According to some embodiments of the present invention, stressing the flow of feeding gas further comprises:
viii) maintaining the flow of the feeding gas at substantially the fifth value of the feeding gas flow for a fourth amount of time; and
ix) after maintaining the flow of the feeding gas at substantially the fifth value, increasing the flow of the feeding gas to a sixth value of the feeding gas flow.

In particular, the sixth value of the feeding gas flow is greater than or equal to the fourth value of feeding gas flow and/or the fourth amount of time is smaller than or equal to the second amount of time.

The fourth value of the feeding gas flow is substantially equal to the sixth value of the feeding gas flow. The sixth value of the feeding gas flow may be comprised between B1 and B2, in particular between B3 and B4, more particularly between B5 and B6.

The sixth value of the feeding gas flow may depend on the fifth value of the feeding gas flow. For instance, the sixth value, *V*₆, of the feeding gas flow may be equal to the product between the aforementioned proportionality constant, *k̅*, and the fifth value, *V*₅, of the feeding gas flow, i.e., *V*₆ = *k̅* × *V*₅.

The fourth amount of time may be substantially equal to the second amount of time. For instance, the fourth amount of time and/or the second amount of time are greater than or equal to C1 and less than or equal to C2, in particular greater than or equal to C3 and less than or equal to C4, more particularly greater than or equal to C5 and less than or equal to C6.

In particular, step viii) is carried out after e.g., immediately after, step vii). Alternatively, or in conjunction with the above, step ix) is carried out immediately after step viii).

Increasing the flow of the feeding gas to the sixth value of the feeding gas flow may be carried out by linearly or stepwise increasing the flow of the feeding in a ninth amount of time, e.g., from the fifth value to the sixth value of the feeding gas flow. The ninth amount of time may be much smaller than the fourth amount of time. For example, the ninth amount of time is less than or equal to a tenth, a hundredth and/or a thousandth of the fourth amount of time.

In some embodiments of the present invention, the first amount of time, the second amount of time, the third amount and/or the fourth amount of time are greater than or equal to a minimum amount of time. In particular, the minimum amount of time may be equal to the average time that occurs to an unreacted molecule e.g., H₂ to travel through the reactor and reach a gas analyzer for analyzing the product gas. The minimum amount of time may be comprised between H1 and H2, in particular between H3 and H4, and, more particularly, between H5 and H6.

According to some embodiments of the present invention, ramping the flow of the feeding gas up comprises, e.g., consists of, increasing the flow rate of the feeding gas from a seventh value of the flow rate of the feeding gas to an eighth value of the flow rate of the feeding gas in a tenth amount of time. In particular, the seventh value and/or the eighth value may be predetermined values of the flow rate of the feeding gas. Alternatively, or in conjunction with the above, the tenth time interval may be a predetermined time interval.

For example, the seventh value of the feeding gas flow are comprised between D1 and D2, in particular between D3 and D4, more particularly between D5 and D6. Moreover, the eighth value of the feeding gas flow may be comprised between E1 and E2, in particular between E3 and E4, more particularly between E5 and E6. For instance, the tenth amount of time is greater than or equal to F1 and less than or equal to F2, in particular greater than or equal to F3 and less than or equal to F4, more particularly greater than or equal to F5 and less than or equal to F6.

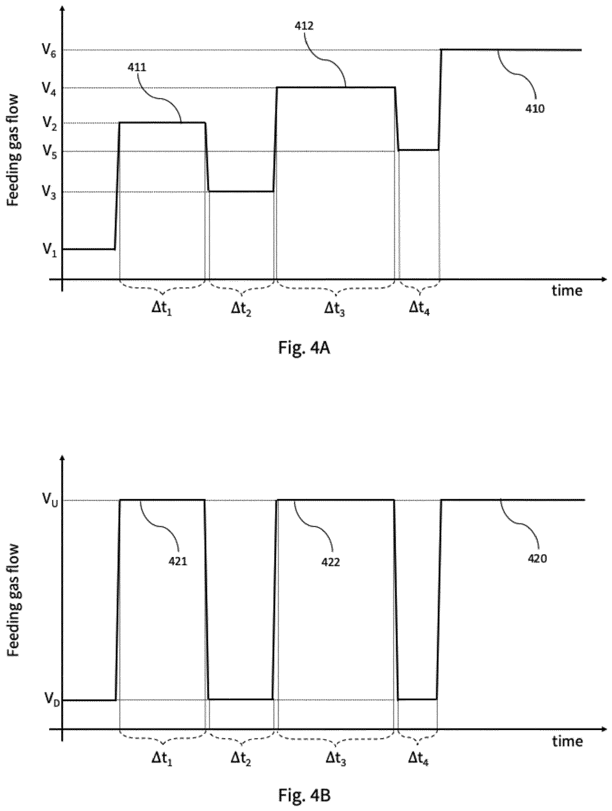

**Figure 4A** schematically depicts the time dependence (solid line 410) of the flow of the feeding gas during stressing according to a first embodiment of the method of the present invention. In particular, the stressing according to the first embodiment of the invention comprises the steps i) to ix) described above. In particular, steps i) to iii) lead to a first peak 411 in the time dependence 410 of the flow of the feeding gas and steps v) to vii) lead to a second peak 412 in the time dependence 410 of the flow of the feeding gas. In this figure, *Vᵢ* is the *i^{th}* value of the feeding gas flow (*i* = 1, 2, 3, 4, 5, 6) and Δ*tⱼ* is the *j^{th}* amount of time (*j* = 1, 2, 3, 4). The fifth value *V*₅ of the feeding gas flow is greater than the third value *V*₃ of the feeding gas flow which, in turn, is greater than the third value *V*₁ of the feeding gas flow, i.e., *V*₅ > *V*₃ > *V*₁. Moreover, the sixth value *V*₆ of the feeding gas flow is greater than the fourth value *V*₄ of the feeding gas flow which, in turn, is greater than the second value *V*₂ of the feeding gas flow, i.e., *V*₆ > *V*₄ > *V*₂.

The second amount of time Δ*t*₂ is greater than the fourth amount of time Δ*t*₄, i.e., Δ*t*₂ > Δ*t*₄, and the third amount of time Δ*t*₃ is greater than the first amount of time Δ*t*₁, that is Δ*t*₃ > Δ*t*₁.

**Figure 4B** schematically depicts the time dependence (solid line 420) of the flow of the feeding gas during stressing according to a second embodiment of the method of the present invention. The stressing according to this embodiment comprises the aforementioned steps i) to ix). Steps i) to iii) lead to the first peak 421 in the time dependence 420 of the flow of the feeding gas and steps v) to vii) lead to the second peak 422 in the time dependence 420 of the flow of the feeding gas.

In this case, the first value *V*₁ of the feeding gas flow is equal to the third and the fifth value of the feeding gas flow, i.e., *V*₁ = *V₃* = *V*₅ = *V_{D}.* The second value *V*₂ of the feeding gas flow is equal to the fourth and the sixth value of the feeding gas flow, i.e., *V*₂ = *V*₄ = *V*₆ = *V_{U}* .

The second amount of time Δ*t*₂ is greater than the fourth amount of time Δ*t*₄ and the third amount of time Δ*t*₃ is greater than the first amount of time Δ*t*₁.

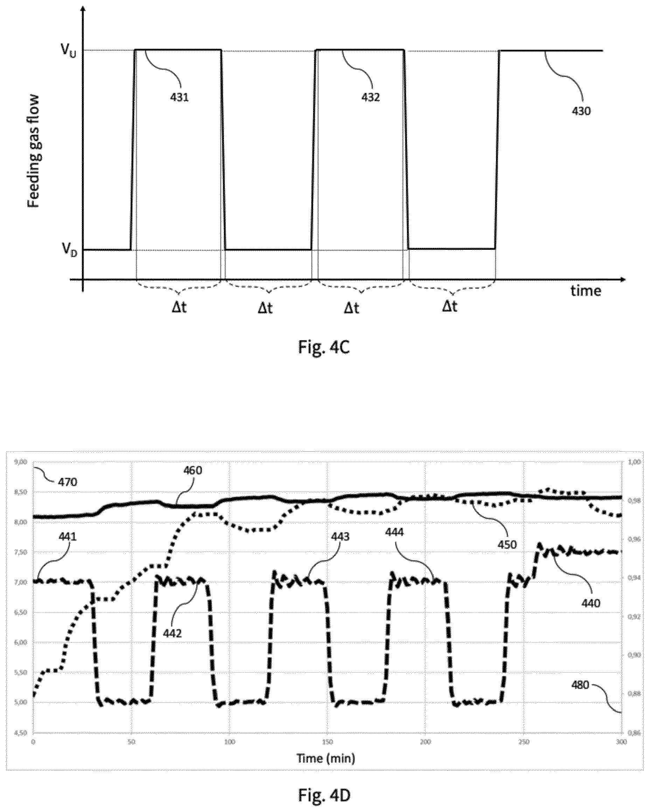

**Figure 4C** schematically depicts the time dependence (solid line 430) of the flow of the feeding gas during stressing according to a third embodiment of the invention, said stressing comprising the steps i) to ix) described above. Steps i) to iii) lead to the first peak 431 in the time dependence 430 of the flow of the feeding gas and steps v) to vii) lead to the second peak 432 in the time dependence 430 of the flow of the feeding gas.

The first value *V*₁ of the feeding gas flow is equal to the third *V*₃ and the fifth *V*₃ value of the feeding gas flow and the second value *V*₂ of the feeding gas flow is equal to the fourth *V*₄ and the sixth value *V*₆ of the feeding gas flow. Moreover, the first amount of time is equal to the second, the third and the fourth amount of time, i.e., Δ*t*₁ = Δ*t*₂ = Δ*t*₃ = Δ*t*₄ = Δ*t*.

It should be understood that the method of the present invention for adapting the biocatalyst may comprise each of the adapting processes individually or may combine the above-described steps of adapting, e.g., preloading the aqueous culture medium with one or more metabolic enhancers, ramping the flow of the feeding gas up, and/or increasing the flow of the feeding gas in steps or one or more intervals of a defined increase regarding the flow of the feeding gas, above described as stressing.

In the context of the present invention, the term "biocatalyst" is defined as and comprises any hydrogenotrophic methanogen suitable to be used alone or in co-culture in a bio-methanation process. In particular, a biocatalyst suitable to be adapted according to the present method belongs to and is selected from the group of Archaea comprising the classes of *Methanobacteria, Methanococci, Methanomicrobia, Methanonatronarchaeia,* and *Methanopyri* each of these classes comprising a number of genera, wherein each genus is divided into families, each family encompassing a large number of known and extensively studied, in the meaning of classified, and unknown, in the meaning of unclassified, species.

According to some embodiments of the present invention, *Methanothermobacter,* and further *Methanothermobacter thermoautotrophicus, Methanothermobacter marburgensis* and/or mixtures thereof, and/or derivatives thereof revealed particularly easy to adapt according to the method of the present invention.

Further, according to some embodiments of the present invention, *Methanothermus fervidus, Methanobrevibacter arboriphilicus, Methanococcus* and *Methanocaldococcus sp.,* and further *Methanocaldococcus bothoordescens, Methanocaldococcus fervens, Methanocaldococcus indicus, Methanocaldococcus infernus, Methanocaldococcus jannaschii, Methanocaldococcus villosus, Methanocaldococcus vulcanius* and/or mixtures thereof, and/or derivatives thereof are particularly suitable to be adapted by the method of the present invention.

As it should be understood from the previous description, the present method is mainly applied and most suitable to improve and stabilize the methanogenesis in a bio-methanation plant, which relies on natural resources regarding for the energy supply. It is particularly important and commercially interesting to stabilize the deployed biocatalyst in the context of an inconsistent or variable supply of natural resources, such as solar energy or wind energy.

It is also important to provide a method for stabilizing the biocatalyst in other e.g., man-made circumstances leading to an inconsistent or variable supply of nutrients or feeding gas. For instance, such circumstances occur when a bio-methanation plant needs to be restarted after being shut down for cleaning and/or maintenance. Restarting a bio-methanation plant using unadapted biocatalysts takes typically about 24 hours.

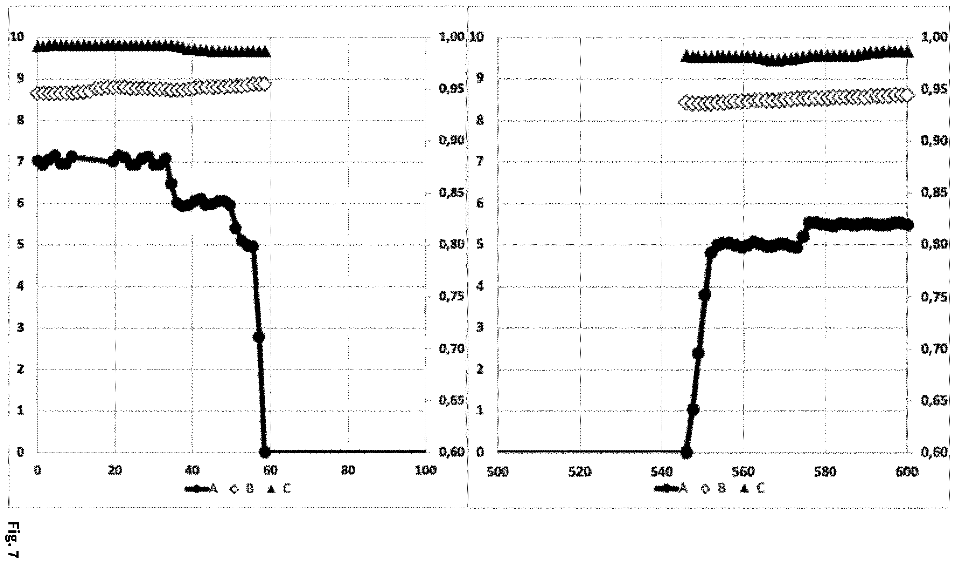

The inventive method of biocatalyst adaptation allows for a substantially quicker restart and allows for reaching a substantially stable methane content in the product gas right after the restart. As is shown on Fig. 7 in a reactor with an adapted biocatalyst a restart after about 8 hours will not lead to an instable methane production but is in less than 10 min up to a grid-quality methane production.

Fig. 7 is additionally interesting as the methane production after the interruption is immediately high and provides grid-quality, namely 95% methane content in the product gas.

### Short description of the figures

**Figure 1** shows impacts on product gas composition, including methane content, of an existing industrial plant during a disturbance.
Fig. 1A depicts the feed gas flow rate of hydrogen, with A = feeding gas (H₂ [kg/h]), and the output gas content (fraction of total) of methane, with C = methane[% of the total gas mixture]; Fig. 1B depicts the corresponding pH value, with B = pH, C = CH₄ [% of the total gas mixture] content; Fig. 1C depicts the consequences of the disturbance on the methane content rate and the total product gas content, with C = CH₄ [% of the total gas mixture], E = H₂ [% of the total gas mixture], F = CO₂ [% of the total gas mixture]; Fig. 1D illustrates the inability for the standard and so-called load-following dosing strategy to recovery by dosing of NH₄OH, with C = CH₄ [% of the total gas mixture], D = NH₄OH [L/h].

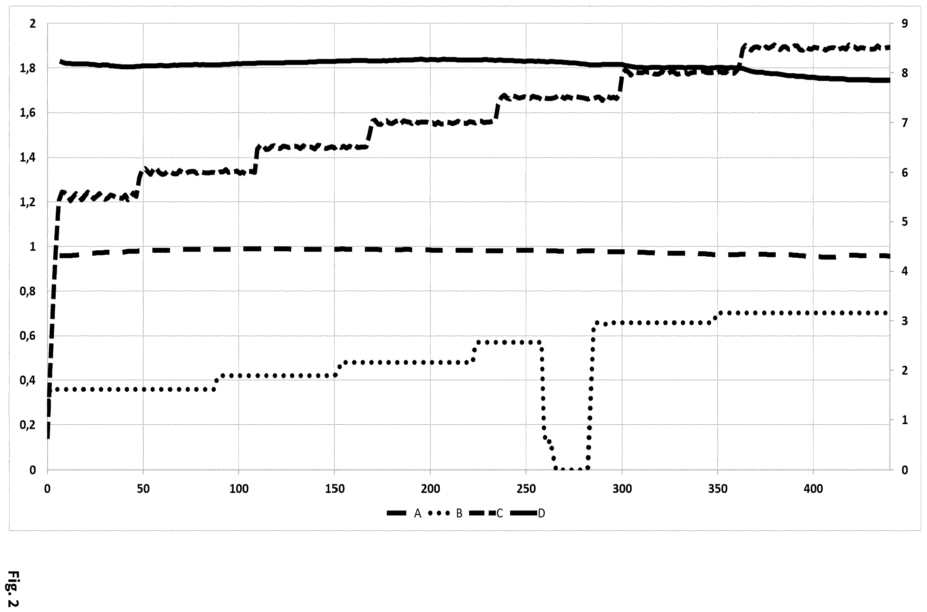

**Figure 2** shows a preloading experiment as described in Example 1, wherein the culture is preloaded with repetitive doses of a redox active chemical, in this case NH₄OH, prior to a substantial increase in reactant gases. In example 1, it is shown that the methane content in the product gas and also the pH value is stable despite a stepwise increase in feeding gases. (y1 = CH₄ [% of the total gas mixture] and NH₄OH [l/h], A = CH₄, B = NH₄OH; y2 = pH and H₂ flow [kg/h], C = H₂ feeding gas, D = pH value; y2; x = run time [min])

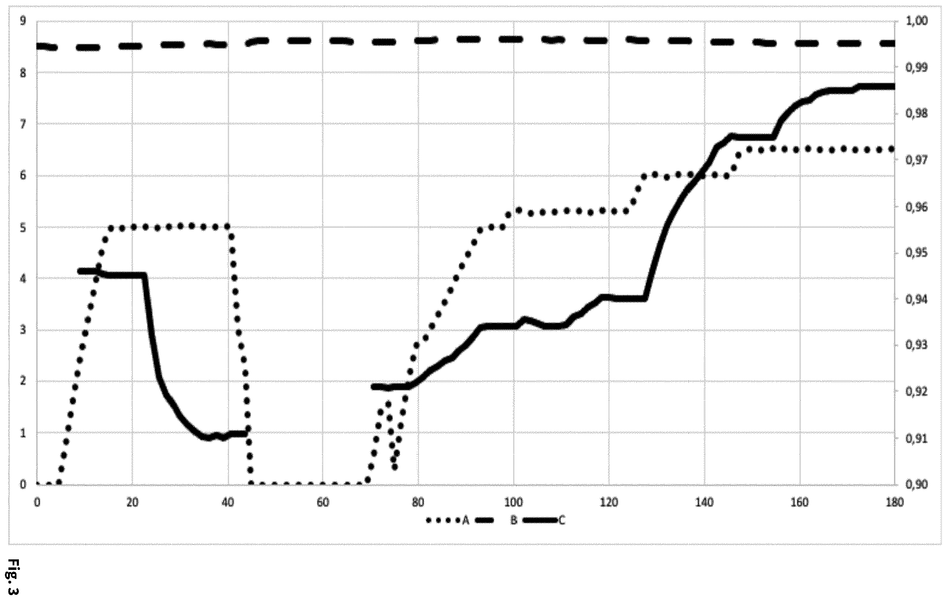

**Figure 3** shows that the sudden increase of the feeding gas causes a crash of the methane production and then, after a short shut down of about 30 min, a careful pulsing and stepwise increase of the flow of the feeding gas allows for a quick initiation of an adaptation, which after only 1,5 hours allows for an even higher input of feeding gas to the now adapted culture. (y1 = pH and H₂ flow [kg/h], A = H₂ feeding gas; B = pH value; y2 = CH₄ [% of the total gas mixture], C = CH₄; x = run time [min])
**Figures 4A, 4B** and **4C** schematically depict the time dependence of the flow of the feeding gas during stressing according to additional embodiments and protocols of the present invention, respectively.
**Figure 4D** depicts the time dependence of the flow of the feeding gas during stressing according to a further embodiment and protocol of the present invention. In Figure 4D, the first ordinate 470 refers to the time dependence 460 of the pH value and to the time dependence 440 of the flow of the feeding gas, the latter time dependence being expressed in kg/h. The second ordinate 480 refers to the time dependence 450 of the relative amount of methane in the product gas. (y1 (470) = pH and H₂ flow [kg/h], 440-444 = H₂ feeding gas; 460 = pH value; 480 = CH₄ [% of the total gas mixture], 450 = CH₄; x = run time [min])

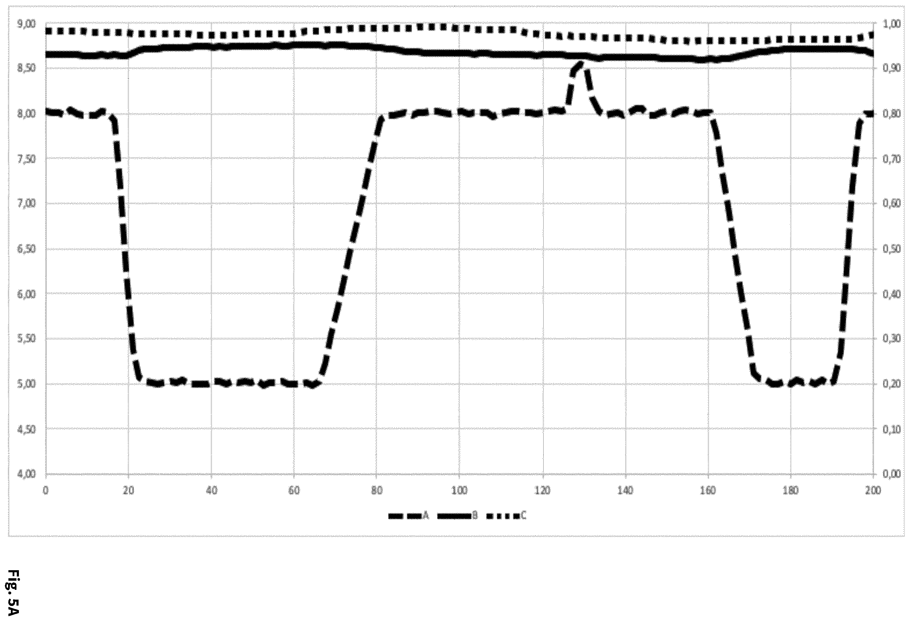

**Figure 5A** shows a flow protocol following a simulated wind profile and thus an energy supply disturbance. Despite such disturbances, which would normally cause a drop in methane content due to higher feed gas flows, in this flow protocol the biocatalyst has already been adapted according to the method of the present invention. Hence, Figure 5A shows that, during the disturbance, the methane content in the product gas is surprisingly stable and constantly provides above 95% of methane in the product gas. (y1 = pH and H₂ flow [kg/h], A = H₂ feeding gas; B = pH value; y2 = CH₄ [% of the total gas mixture], C = CH₄; x = run time [min])

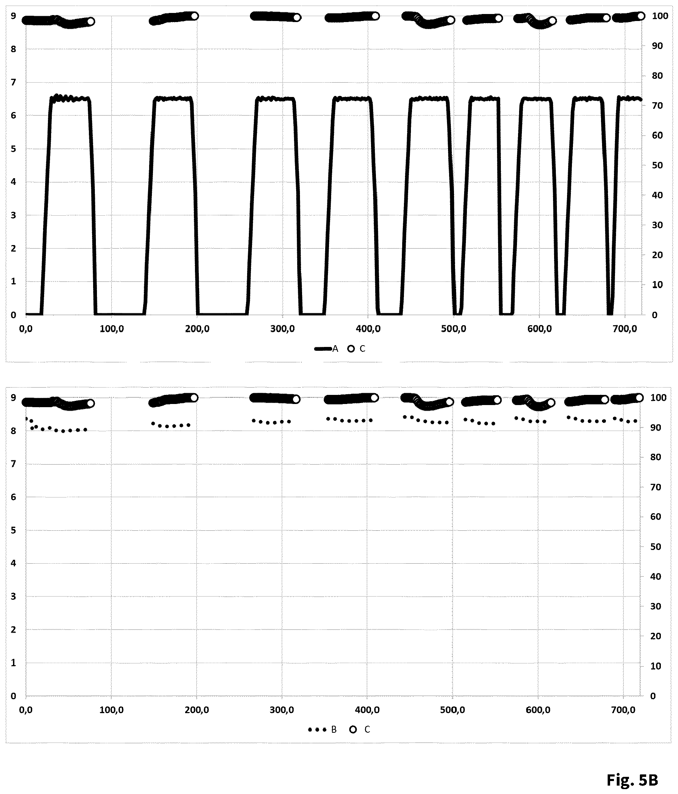

**Figure 5B** shows the same surprising effect of the method of the invention but with an extreme challenge for the system. The adapted and stimulated biocatalyst keeps the methane content up and stably above 95 %, despite the sudden variations of the feed flow of the feeding gas which varies repeatedly between 0 kg/h and 6,5 kg/h. (y1 = pH and H₂ flow [kg/h], A = H₂ feeding gas, B = pH; y2 = CH₄ [% of the total gas mixture], C = CH₄; x = run time [min]).

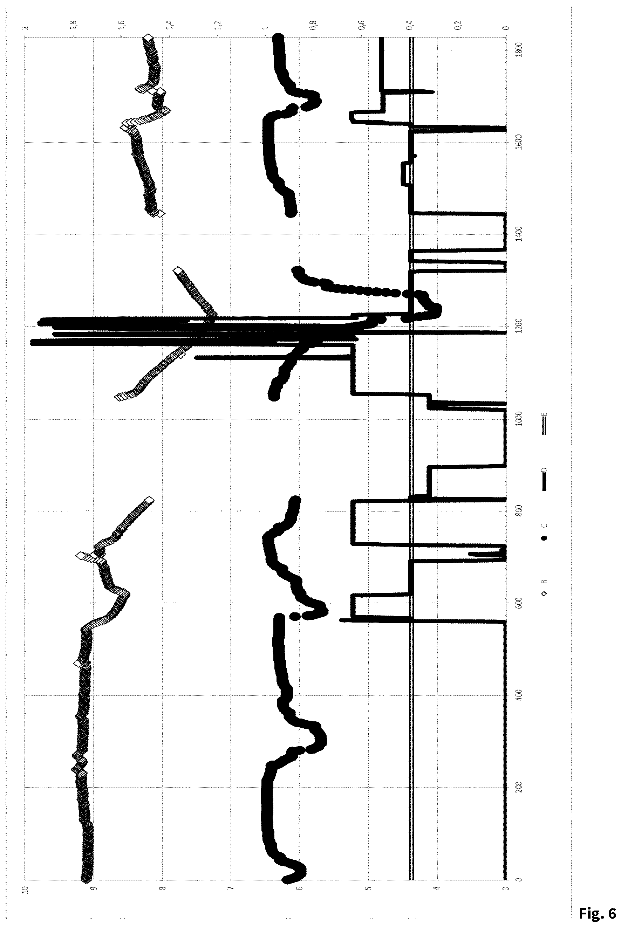

**Figure 6** shows a crash of the methane content in a reactor employing an unadapted biocatalyst as well as the attempt to immediately after the crash to increase the NH₄OH dosing. However, as can be seem in the flow chart neither the addition of NH₄OH nor the interruption of the feeding gas flow can recover the system quickly. A slow stabilization can be seen earliest after about 1000 minutes. (y1 = pH and H₂ flow [kg/h], A = H₂ feeding gas; B = pH value; y2 = CH₄ [% of the total gas mixture] and NH₄OH [L/h], C = CH₄, D = NH₄OH; x = run time [min]).
**Figure 7** shows the flow protocol of a maintenance shut down in a reactor with an adapted biocatalyst as well the successful and immediate restart after about 9 hours.

### Examples

The following examples illustrate viable ways of carrying out the described method as intended, without the intent of limiting the invention to said examples.

All of the following examples have been carried out elaborated in an industrial-sized non-commercial methanation plant. The present examples have been carried out in a Continuous Stir Tank Reactor (CSTR) which contains up to a volume of at least 3.200 liters of suitable cell culture medium. This choice of the type of methanation reactor, however, is not limiting and other types of methanation reactors may be used to carry out the following examples. The medium comprises a biocatalyst, typically a hydrogenotrophic methanogens, belonging to the archaea. The reactant gases H₂ and CO₂ are mixed as well as optionally compressed and preheated prior to mixing with the culture medium in the reactor.

On all the Figures presenting results only the H₂ flow rate is indicated, which however stands for the influx of the feeding gases including H₂ and CO₂ under ideal and non-limiting production conditions. Hence, in this case the methanation reactor is fed with between 5,0 and 5,5 kg/h of H₂ and about 27,8 kg/h of CO₂. Similarly, under these ideal and non-limiting production conditions, the nominal amount of the nitrogen source is kept at about 0,4 g/L NH₄OH.

Any disturbance of such ideal conditions leads to a loss in the methane content. In particular, Figures 1A to 1D show how the lack of consistency (e.g., a sudden increase) in the feeding gas influx (cf. Fig.1A) leads to a very instable methane content (Fig.1C). In particular, the methane produced in the reactor amounts to only about 30% of the product gas. Furthermore, as shown in Fig. 1B, the instability in the feeding gas influx leads to an instability of the pH value of the culture medium.

The present invention provides methods to adapt the biocatalyst for expected or unexpected variations in the feeding gas influx and/or in the energy supply availability and provides for or guarantees a stable methane content despite such inconsistencies.

### Example 1 - Preloading

In this Example it is shown how a chemical adaptation of the biocatalyst improves the stability of the methanation process and the methane content. For this, the biocatalyst is adapted by preloading the culture medium with a metabolic enhancer, namely NH₄OH.

The preloading with NH₄OH started only shortly before or around 0,5 hours prior to a planned increase in feeding gas. In particular, the preloading was carried out by introducing a plurality of doses of NH₄OH into the aqueous culture medium in the methanation reactor. Each dose of the plurality of doses was introduced in the culture medium at a respective point in time. These points in time were distributed in a substantially homogenous way. In particular, in this Example, the dosing took place about every 60 minutes and the individual dose of NH₄OH was around 0,97 mol -N. Ultimately, said plurality of doses of NH₄+ did sum up to 5,03 mol -N in the culture medium

The feed flow of the feeding gas was increased always shortly after the dosing with NH₄OH, in this Example 30 min after the dosing. In total, the feed flow of the feeding gas was increased from a starting value of 5.5kg/h to a value of 8,5 kg/h over a period of 7 hours, without any crashes or drops in the methane production rate.

As can be seen in Fig. 2, the methane content was kept stable despite the relatively swift increase in the feed flow of the feeding gas influx. Notably, also the pH value remained substantially stable. As mentioned above, the stability of the pH value provides a further indication of the biocatalyst's capability to fully compensate for the increased amount of reactant gas and to fully convert said gas into methane.

### Example 2 - Ramping

In this Example, it is shown how "ramping up" the gas flow triggers the physical adaptation of the biocatalyst, thereby improving the stability of the methanation process and the increasing methane production at high methane content in the product gas.

In this Example, a stepwise increase of the feed flow of the feeding gas flow was applied to a system that has crashed due to an unexpected high feeding gas influx into an unadapted biocatalyst culture. As shown in Figure 3, the sudden increase of feeding gas did cause a crash of the methane content in the product gas and then, after a short shut down of about 30 min, a careful pulsing and stepwise increase of the feed flow of the feeding gas allowed for a quick initiation of an adaptation, which, after only 1,5 hours, allowed for an even higher input of feeding gas to the now adapted culture.

Interestingly, the OD610 of the respective biocatalyst reaction vessel was prior to the preloading, during the preloading and even hours after the increase in feeding gas in this Example around OD45.

### Example 3 - Stressing

In this Example, it is demonstrated how stressing the feed flow of the feeding gas- leads to an adapted biocatalyst and thereby improves the stability of the methane content in the product gas.

Unfortunately, it is often found that a sudden increase of feeding gas typically causes a crash of the system, which then may be recovered by adding nutrients and/or by reducing the feeding gas influx (cf. Fig 6). After a sudden feeding gas input of around 7,5 kg/h the systems crashed, and the feeding gas was reduced to the normal input of 5 kg/h.

In this Example, the inventors showed that, as a response to such a sudden increase or to a system crash causing a substantial reduction in the methane content in the product gas, the stressing of the feed flow of the feeding gas initiates the adaptation of the biocatalyst and stabilizes the production of methane.

Figure 4D depicts the time dependence (dashed line 440) of the flow of the feeding gas after the crash, during stressing. This figure shows also the effect of the stressing on the time dependence (dotted line 450) of the amount of methane in the product gas and on the time dependence (solid line 460) of the pH value of the culture medium. In Figure 4D, the first ordinate 470 refers to the time dependence 460 of the pH value and to the time dependence 440 of the flow of the feeding gas, the latter time dependence being expressed in kg/h. The second ordinate 480 refers to the time dependence 450 of the relative amount of methane in the product gas.

The stressing comprised the steps i) to iii) described above which led to the first spike 441, in which the flow of the feeding gas was increased from about 5 kg/h to about 7 kg/h and maintained at about 7 Kg/h for 30 min. As shown in Figure 4D, already the first spike 441 leads to an increase in the amount of methane in the product gas. The stressing comprised also steps v) to vii) which led to the second spike 442, which, as shown in Figure 4D, is similar in shape to the first one. After the second spike 442, the methane production is up to grid quality and, thus, more than amount of methane in the product gas is above 95%. The stressing led to two Figure 4D two further spikes 443, 444 which further stabilizes the methane content at higher feed gas flows. After about 250 min, the flow of the feeding gas was increased to 7,5 kg/h, i.e., to the amount leading to the failure of the unadapted biocatalyst. As shown in Figure 4D, stressing allows for a sudden increase of the flow of the feeding gas of at least 50%, in this case from 5 kg/h to 7,5 kg/h.

Multiple stressing protocols have been tested and were found to lead to the similar result. These protocols differ from one another in the duration, in the number, in the frequency, and/or in the amplitude of the spikes and lead a biocatalyst adaptation. Exemplary time dependences of such alternative protocols are schematically depicted as Figures 4A-C.

### Example 4 - Simulations with adapted biocatalysts

A biocatalyst culture, which has been adapted and/or stimulated as in Examples 1 to 3 keeps a stable methane content in the product gas even under challenging and changing gas flow situations. Such challenging situations have been simulated for the flow protocol shown in Figure 5A, which illustrates the volatile energy availability and, thus, the jumping H₂ availability in a typical wind profile. In particular, it is shown that an adapted biocatalyst culture leads to a substantially stable methane production and that the stimulated biocatalyst keeps the methane content in the product gas up and also converts the provided feeding gas fast enough to not even cause a change in pH value.

Figure 5B shows the same surprising effects of the method of the invention but with an extreme challenge for the system. The adapted and stimulated biocatalyst keeps the methane production up and stably above 95 %, despite the sudden variations of the feed flow of the feeding gas which varies repeatedly between 0 kg/h and 6,5 kg/h.

### Example 5 - combination of the preloading and stressing strategy

In this Example we successfully tried to increase the unexpected gas flow to up to 200% above the initial flow, triggered e.g. by excess electricity of 200%.

For this the established biocatalyst culture was adapted firstly by preloading the culture with NH₄OH for 5 dosing steps, each dosing of NH₄OH was 60 min later followed by an increase of feeding gas of 20%. After a short window to measure the stability of the methane content and confirming the grid quality, namely a methane content of at least 95% in the product gas, a second round of adaptation was started.

This second round was a stressing protocol with feeding gas spikes, which increased the volume by 20% for 10 minutes, followed by a 10 min period of reducing the gas feed to the lower level. The stressing protocol is selected from the protocols as demonstrated in Figure 4A or 4B. After 3 spikes the feeding gas was kept stable on the raised (+20%) level for a short window to measure the stability of the methane content and confirming the grid quality, namely a methane content of at least 95% in the product gas. Typically, the control windows are about 1 hour.

Thereafter the next round of adaptation was started and again was used a stressing protocol with feeding gas spikes, which increased the volume by 20% for 10 minutes, followed by a 10 min period of reducing the gas feed to a lower level. The stressing protocol is selected from the protocols as demonstrated in Fig. 4A or 4B.

After 3 spikes the feeding gas was kept stable on the raised (additional +20%) level for a short window to measure the stability of the methane content in the product gas and confirming the grid quality, namely a methane content of at least 95% in the product gas. This stressing protocol was repeated for 5 times until the adapted biocatalyst was capable of transforming the additional 200% of electricity (i.e. as additional feeding gas input) in to methane and over the whole time substantially produced a product gas with around or above 95% methane content.

### References

Thauer et al., 2008, Nature review Microbiology, Vol 6, pp 579-591
Matin et al., 2013, Archaea, Article ID 157529, 11 pages
Thema et al., 2019, Energies 2019, 12, 1670

## Claims

1. Method for stabilizing methanogenesis of a biocatalyst despite influx variations of a flow of a feeding gas and/or variations of the energy supply in form of the supply of a feeding gas for bio-methanation, the method comprising the steps of:
- physically and/or chemically adapting the biocatalyst responsible for the methanation in a bio-methanation plant comprising at least one of the following adaptations steps selected from the group of
• loading an aqueous culture medium with a dose of one or more metabolic enhancers, the aqueous culture medium comprising the biocatalyst and being comprised in the reactor;
• ramping the flow of the feeding gas up, and/or
• stressing the flow of the feeding gas at least by sudden increasing the flow of the feeding gas by a defined increase;
- and continuously measuring the methane production or methane content in the product gas.

2. Method according to claim 1, wherein the step of chemically adapting the biocatalyst comprises the loading of the culture medium with one or more metabolic enhancers, which starts between about 2 hour and 1 minute prior to the influx variations of the energy supply in form of the supply of a feeding gas for bio-methanation or to the increase in feeding gas flow.

3. Method according to claims 1 or 2, wherein the loading comprises an increase of the amount of the one or more metabolic enhancers selected from the group of redox active chemicals comprising amino acids, carbonate solutions, pH changing solutions, Na₂S, NH₄OH and combinations of any or several thereof.

4. Method according to claims 1 to 3, wherein the loading comprises a singular step of adding the one or more metabolic enhancers or a plurality of steps for adding the one or more metabolic enhancers.

5. Method according to any one of claims 1 to 4, wherein the step of physical adapting the biocatalyst comprises ramping the flow of the feeding gas up and/or stressing the flow of feeding gas, and wherein said steps of adapting the biocatalyst start between 5 hours and 0,5 hours prior to the influx variations of the energy supply in form of the supply of a feeding gas for bio-methanation or to the increase in feeding gas flow.

6. Method according to any one of claims 1 to 5, wherein stressing the flow of feeding gas comprises:
i. increasing the flow of the feeding gas from a first value of the feeding gas flow to a second value of the feeding gas flow;
ii. maintaining the flow of the feeding gas at the second value of the feeding gas flow for a first amount of time; and
iii. after maintaining the flow of the feeding gas at the second value of the feeding gas flow, decreasing the flow of the feeding gas to a third value of feeding gas flow,
wherein the third value of the feeding gas flow is greater than or equal to the first value of feeding gas flow.

7. Method according to claim 6, wherein stressing the flow of feeding gas further comprises:
iv. maintaining the flow of the feeding gas at the third value of feeding gas flow for a second amount of time;
v. after maintaining the flow of the feeding gas at the third value, increasing the flow of the feeding gas to a fourth value of the feeding gas flow;
vi. maintaining the flow of the feeding gas at the fourth value of feeding gas flow for a third amount of time; and
vii. after maintaining the flow of the feeding gas at the fourth value, decreasing the flow of the feeding gas to a fifth value of feeding gas flow,
wherein the fifth value of the feeding gas flow is greater than or equal to the third value of feeding gas flow, the fourth value of the feeding gas flow is greater than or equal to the second value of feeding gas flow, and/or the third amount of time is greater than or equal to the first amount of time.

8. Method according to claim 7, wherein stressing the increase the flow of feeding gas comprises:
viii. maintaining the flow of the feeding gas at the fifth value of the feeding gas flow for a fourth amount of time; and
ix. after maintaining the flow of the feeding gas at the fifth value, increasing the flow of the feeding gas to a sixth value of the feeding gas flow,
wherein the sixth value of the feeding gas flow is greater than or equal to the fourth value of feeding gas flow and/or the fourth amount of time is smaller than or equal to the second amount of time.

9. Method according to any one of claims 1 to 8, wherein the step of adapting the biocatalyst comprises loading the aqueous culture medium, ramping the flow of the feeding gas up, and stressing the flow of the feeding gas at least one interval of a defined increase regarding the flow of the feeding gas.

10. Method according to any of the preceding claims, wherein the biocatalyst comprises or consists of a hydrogenotrophic methanogen.

11. Method according to any of the preceding claims, wherein the biocatalyst is selected from the class of *Methanobacteria, Methanococci, Methanomicrobia, Methanonatronarchaeia,* and *Methanopyri* or combinations thereof.

12. Use of the method according to any of the preceding claims for stabilizing methanogenesis of a biocatalyst after maintenance interruption of a bio-methanation plant, the bio-methanation plant using the biocatalyst for the production of methane.

13. Use of the method according to any of claims 1 to 12 for balancing inconsistent energy supply from renewable energy sources needed for the supply of a feeding gas for bio-methanation to a bio-methanation plant, the bio-methanation plant using a biocatalyst for the production of methane.

## Patentansprüche

1. Verfahren zur Stabilisierung der Methanogenese eines Biokatalysators trotz Zuflussschwankungen eines Flusses eines Speisegases und/oder Schwankungen der Energiezufuhr in Form der Zufuhr eines Speisegases für die Biomethanisierung, wobei das Verfahren die folgenden Schritte umfasst:
- physikalisches und/oder chemisches Anpassen des für die Methanisierung verantwortlichen Biokatalysators in einer Biomethanisierungsanlage, das mindestens einen der folgenden Anpassungsschritte umfasst, die aus der folgenden Gruppe ausgewählt sind:
· Beladen eines wässrigen Kulturmediums mit einer Dosis eines oder mehrerer Stoffwechselverstärker, wobei das wässrige Kulturmedium den Biokatalysator enthält und in den Reaktor eingebracht ist;
· Erhöhen des Flusses des Speisegases und/oder
· Beanspruchen des Flusses des Speisegases mindestens durch plötzliches Erhöhen des Flusses des Speisegases um eine definierte Erhöhung;
- und kontinuierliches Messen der Methanproduktion oder des Methangehalts im Produktgas.

2. Verfahren nach Anspruch 1, wobei der Schritt des chemischen Anpassens des Biokatalysators das Beladen des Kulturmediums mit einem oder mehreren Stoffwechselverstärkern umfasst, das zwischen etwa 2 Stunden und 1 Minute vor den Zuflussschwankungen der Energiezufuhr in Form der Zufuhr eines Speisegases für die Biomethanisierung oder vor der Erhöhung des Speisegasflusses beginnt.

3. Verfahren nach Anspruch 1 oder 2, wobei das Beladen eine Erhöhung der Menge des einen oder der mehreren Stoffwechselverstärker umfasst, die aus der Gruppe der redoxaktiven Chemikalien ausgewählt sind, welche Aminosäuren, Karbonatlösungen, pHverändernde Lösungen, Na₂S, NH₄OH und Kombinationen eines oder mehrerer davon umfasst.

4. Verfahren nach einem der Ansprüche 1 bis 3, wobei das Beladen einen einzelnen Schritt der Zugabe des einen oder der mehreren Stoffwechselverstärker oder eine Vielzahl von Schritten zur Zugabe des einen oder der mehreren Stoffwechselverstärker umfasst.

5. Verfahren nach einem der Ansprüche 1 bis 4, wobei der Schritt des physikalischen Anpassens des Biokatalysators ein Herauffahren des Flusses des Speisegases und/oder ein Beanspruchen des Flusses des Speisegases umfasst und wobei die Schritte des Anpassens des Biokatalysators zwischen 5 Stunden und 0,5 Stunden vor den Zuflussschwankungen der Energiezufuhr in Form der Zufuhr eines Speisegases für die Biomethanisierung oder vor der Erhöhung des Speisegasflusses beginnen.

6. Verfahren nach einem der Ansprüche 1 bis 5, wobei das Beanspruchen des Flusses des Speisegases umfasst:
i. Erhöhen des Flusses des Speisegases von einem ersten Wert des Speisegasflusses auf einen zweiten Wert des Speisegasflusses;
ii. Aufrechterhalten des Flusses des Speisegases auf dem zweiten Wert des Speisegasflusses für eine erste Zeitperiode und
iii. nach dem Aufrechterhalten des Flusses des Speisegases auf dem zweiten Wert des Speisegasflusses, Verringern des Flusses des Speisegases auf einen dritten Wert des Speisegasflusses,
wobei der dritte Wert des Speisegasflusses größer als der oder gleich dem ersten Wert des Speisegasflusses ist.

7. Verfahren nach Anspruch 6, wobei die Beanspruchung des Flusses des Speisegases ferner umfasst:
iv. Aufrechterhalten des Flusses des Speisegases auf dem dritten Wert des Speisegasflusses für eine zweite Zeitperiode;
v. nach dem Aufrechterhalten des Flusses des Speisegases auf dem dritten Wert, Erhöhen des Flusses des Speisegases auf einen vierten Wert des Speisegasflusses;
vi. Aufrechterhalten des Flusses des Speisegases auf dem vierten Wert des Speisegasflusses für eine dritte Zeitperiode und
vii. nach dem Aufrechterhalten des Flusses des Speisegases auf dem vierten Wert, Verringern des Flusses des Speisegases auf einen fünften Wert des Speisegasflusses,
wobei der fünfte Wert des Speisegasflusses größer als der oder gleich dem dritten Wert des Speisegasflusses ist, der vierte Wert des Speisegasflusses größer als der oder gleich dem zweiten Wert des Speisegasflusses ist und/oder die dritte Zeitperiode größer als die oder gleich der ersten Zeitperiode ist.

8. Verfahren nach Anspruch 7, wobei das Beanspruchen der Erhöhung des Flusses des Speisegases umfasst:
viii. Aufrechterhalten des Flusses des Speisegases auf dem fünften Wert des Speisegasflusses für eine vierte Zeitperiode und
ix. nach dem Aufrechterhalten des Flusses des Speisegases auf dem fünften Wert, Erhöhen des Flusses des Speisegases auf einen sechsten Wert des Speisegasflusses,
wobei der sechste Wert des Speisegasflusses größer als der oder gleich dem vierten Wert des Speisegasflusses ist und/oder die vierte Zeitperiode kleiner als die oder gleich der zweiten Zeitperiode ist.

9. Verfahren nach einem der Ansprüche 1 bis 8, wobei der Schritt des Anpassens des Biokatalysators das Beladen des wässrigen Kulturmediums, das Herauffahren des Flusses des Speisegases und das Beanspruchen des Speisegasflusses in mindestens einem Intervall einer definierten Erhöhung mit Bezug auf den Fluss des Speisegases umfasst.

10. Verfahren nach einem der vorhergehenden Ansprüche, wobei der Biokatalysator ein hydrogenotrophes Methanogen umfasst oder aus einem solchen besteht.

11. Verfahren nach einem der vorhergehenden Ansprüche, wobei der Biokatalysator aus der Klasse der *Methanobacteria, Methanococci, Methanomicrobia, Methanonatronarchaeia* und *Methanopyri* oder Kombinationen davon ausgewählt ist.

12. Verwendung des Verfahrens nach einem der vorhergehenden Ansprüche zur Stabilisierung der Methanogenese eines Biokatalysators nach einer Wartungsunterbrechung einer Biomethanisierungsanlage, wobei die Biomethanisierungsanlage den Biokatalysator für die Produktion von Methan verwendet.

13. Verwendung des Verfahrens nach einem der Ansprüche 1 bis 12 zum Ausgleich einer uneinheitlichen Energiezufuhr aus erneuerbaren Energiequellen, die für die Zufuhr eines Speisegases für die Biomethanisierung zu einer Biomethanisierungsanlage benötigt wird, wobei die Biomethanisierungsanlage einen Biokatalysator für die Produktion von Methan verwendet.

## Revendications

1. Méthode en vue de la stabilisation de la méthanogénèse d'un biocatalyseur malgré les variations d'afflux d'un flux d'un gaz d'alimentation et/ou les variations de distribution d'énergie sous la forme de l'approvisionnement d'un gaz d'alimentation destiné à la biométhanisation, la méthode comprenant les étapes suivantes :
- adaptation physique et/ou chimique du biocatalyseur responsable de la méthanisation dans une installation de biométhanisation comprenant au moins une des étapes d'adaptation suivantes sélectionnée à partir du groupe de
· chargement d'un milieu de culture aqueux avec une dose d'un ou de plusieurs activateurs métaboliques, le milieu de culture aqueux comprenant le biocatalyseur et étant placé dans le réacteur ;
· augmentation du flux du gaz d'alimentation, et/ou
· mise sous contrainte du flux du gaz d'alimentation au moins par une augmentation soudaine du flux du gaz d'alimentation d'une valeur définie ;
- et mesure en continu de la production de méthane ou de la teneur en méthane dans le gaz de production.

2. Méthode selon la revendication 1, dans laquelle l'étape d'adaptation chimique du biocatalyseur comprend le chargement du milieu de culture d'un ou plusieurs activateurs métaboliques, qui démarre entre environ 2 heures et 1 minute avant les variations d'afflux de l'approvisionnement d'énergie sous la forme de l'approvisionnement d'un gaz d'alimentation en vue de la biométhanisation ou avant l'augmentation en flux de gaz d'alimentation.

3. Méthode selon les revendications 1 ou 2, dans laquelle le chargement comprend une augmentation du volume des un ou plusieurs activateurs métaboliques sélectionnés à partir du groupe de substances chimiques actives d'oxydoréduction comprenant les acides aminés, les solutions de carbonate, les solutions de modification du pH, le Na₂S, le NH₄OH et des combinaisons d'un ou plusieurs de ceux-ci.

4. Méthode selon la revendication 3, dans laquelle le chargement comprend une étape singulière d'addition des un ou plusieurs activateurs métaboliques ou une pluralité d'étapes en vue de l'addition des un ou plusieurs activateurs métaboliques.

5. Méthode selon une quelconque des revendications 1 à 4, dans laquelle l'étape d'adaptation physique du biocatalyseur comprend l'augmentation du flux du gaz d'alimentation, et/ou la mise sous contrainte du flux du gaz d'alimentation, et dans laquelle lesdites étapes d'adaptation du biocatalyseur démarrent entre 5 heures et 0,5 heure avant les variations d'afflux de l'approvisionnement d'énergie sous la forme de l'approvisionnement d'un gaz d'alimentation en vue de la biométhanisation ou avant l'augmentation en flux de gaz d'alimentation.

6. Méthode selon une quelconque des revendications 1 à 5, dans laquelle la mise sous contrainte du flux de gaz d'alimentation comprend :
i. l'augmentation du flux du gaz d'alimentation à d'une première valeur du flux de gaz d'alimentation à une seconde valeur du flux de gaz d'alimentation ;
ii. le maintien du flux du gaz d'alimentation à la seconde valeur du flux de gaz d'alimentation pendant une première quantité de temps ; et
iii. après le maintien du flux du gaz d'alimentation à la seconde valeur du flux du gaz d'alimentation, la réduction du flux du gaz d'alimentation à une troisième valeur de flux de gaz d'alimentation,
dans laquelle la troisième valeur du flux de gaz d'alimentation est supérieure ou égale à la première valeur de flux de gaz d'alimentation.

7. Méthode selon la revendication 6, dans laquelle la mise sous contrainte du flux de gaz d'alimentation comprend :
iv. le maintien du flux du gaz d'alimentation à la troisième valeur de flux de gaz d'alimentation pendant une seconde quantité de temps ;
v. après le maintien du flux du gaz d'alimentation à la troisième valeur, l'augmentation du flux du gaz d'alimentation à une quatrième valeur de flux de gaz d'alimentation ;
vi. le maintien du flux du gaz d'alimentation à la quatrième valeur de flux de gaz d'alimentation pendant une troisième quantité de temps ;
vii. après le maintien du flux du gaz d'alimentation à la quatrième valeur, la réduction du flux du gaz d'alimentation à une cinquième valeur de flux de gaz d'alimentation,
dans laquelle la cinquième valeur du flux de gaz d'alimentation est supérieure ou égale à la troisième valeur de flux de gaz d'alimentation, la quatrième valeur du flux de gaz d'alimentation est supérieure ou égale à la seconde valeur de flux de gaz d'alimentation et/ou la troisième quantité de temps est supérieure ou égale à la première quantité de temps.

8. Méthode selon la revendication 7, dans laquelle la mise sous contrainte de l'augmentation du flux de gaz d'alimentation comprend :
viii. le maintien du flux du gaz d'alimentation à la cinquième valeur du flux de gaz d'alimentation pendant une quatrième quantité de temps ; et
ix. après le maintien du flux du gaz d'alimentation à la cinquième valeur, l'augmentation du flux du gaz d'alimentation à une sixième valeur du flux du gaz d'alimentation,
dans laquelle la sixième valeur du flux de gaz d'alimentation est supérieure ou égale à la quatrième valeur de flux de gaz d'alimentation et/ou la quatrième quantité de temps est inférieure ou égale à la seconde quantité de temps.

9. Méthode selon une quelconque des revendications 1 à 8, dans laquelle l'étape d'adaptation du biocatalyseur comprend le chargement du milieu de culture aqueux, l'augmentation du flux du gaz d'alimentation et la mise sous contrainte du flux du gaz d'alimentation à au moins un intervalle d'une augmentation définie concernant le flux du gaz d'alimentation.

10. Méthode selon une quelconque des revendications précédentes, dans laquelle le biocatalyseur comprend ou se compose d'un méthanogène hydrogénotrophe.

11. Méthode selon une quelconque des revendications précédentes, dans laquelle le biocatalyseur est sélectionné à partir de la classe de *Methanobacteria,* de *Methanococci, Methanomicrobia,* de *Methanonatronarchaeia,* et de *Methanopyri* ou de combinaisons de ceux-ci.

12. Utilisation de la méthode selon une quelconque des revendications précédentes en vue de la stabilisation de la méthanogénèse d'un biocatalyseur après une interruption de maintenance d'une installation de biométhanisation, l'installation de biométhanisation utilisant le biocatalyseur en vue de la production de méthane.

13. Utilisation de la méthode selon une quelconque des revendications 1 à 12 en vue de l'équilibrage d'approvisionnement irrégulier d'énergie provenant de sources d'énergie renouvelables nécessitées en vue de l'approvisionnement d'un gaz d'alimentation en vue de la biométhanisation vers une installation de biométhanisation, l'installation de biométhanisation utilisant un biocatalyseur en vue de la production de méthane.
